# EUROPEAN PATENT SPECIFICATION

(11) **EP 0 724 430 B1**
(45) Date of publication and mention of the grant of the patent: **08.09.1999**
(21) Application number: 92908209.7
(22) Date of filing: 15.04.1992
(51) Int. Cl.: A61K 9/127

(54) **METHOD FOR THE FORMATION OF LIPOSOMES AND COMPOSITIONS FOR USE THEREIN**
METHODE ZUR BILDUNG VON LIPOSOMEN UND ZUSAMMENSETZUNGEN ZUR DEREN HERSTELLUNG
PROCEDE DE FORMATION DE LIPOSOMES ET COMPOSITIONS UTILISEES DANS LE CADRE DE CE PROCEDE

(30) Priority: 16.04.1991 GB 91080432
(43) Date of publication of application: 07.08.1996
(73) Proprietor: PHARES PHARMACEUTICAL RESEARCH N.V., Curacao Netherlands Antilles (AN)
(72) Inventor: LEIGH, Steven, Bishop's Walk, Croydon CRO 5BA (GB)
(74) Representative: Gaunt, Robert John
(86) International application number: GB9200691
(87) International publication number: WO9218103

(56) References cited:
- EP-A- 0 158 441
- GB-A- 2 089 681
- US-A- 4 235 871
- US-A- 4 522 803

## Description

This invention relates to a method of forming liposomes or related bilayered structures and to a composition for use in such a method. The method is particularly suited to the formation of liposomes in situ on biological surfaces.

Liposomes are lipid vesicles made up of alternating bilayers separated by aqueous spaces. Bilayered structures are extended structures which may or may not be vesicular, but are made up of lipid bilayers separated by aqueous spaces. Early interest in liposomes was confined to role models for cell membranes. However, there is now considerable interest in the pharmaceutical and cosmetic use of liposomes as carriers. Biologically active compounds entrapped in liposomes are protected from the external environment and diffuse out gradually to give a sustained effect. Despite the many advantages of liposomes, however, low entrapment, production and stability problems have hindered their practical use as carriers of biologically active compounds. Numerous methods for dealing with these problems have been reported.

Previous techniques for forming liposomes generally rely on solvent evaporation or high energy processing. The most basic method involves spreading a thin film of lipid in a flask by removal of the solvent which is usually chloroform. Multilamellar vesicles (MLV's) are formed by sonicating an aqueous dispersion of the lipid film. In an improvement on this method, US Patent No. 4235871 describes reversephase evaporation vesicles (REV's). In this disclosure, an aqueous medium is added to a solution of lipid in diethyl ether to obtain a dispersed phase. After sonication to a homogeneous dispersion, the solvent is evaporated under reduced pressure to yield a suspension of liposomes.

US Patent No. 4522803 claims stable plurilamellar vesicles (SPLV's). They are prepared in somewhat similar fashion to the REV's of US Patent No. 4235871. A smaller quantity of aqueous medium is added to the lipid solution to ensure formation of intermediate emulsion droplets. After or during sonication, the solvent is removed in a stream of nitrogen. Further aqueous solution is added to give a liposome suspension.

GB-A-2089681 describes a process for making lipid membrane structures, wherein a solution of bilayer forming compounds in a two component solvent system (i.e. a combination of water-miscible and water-immiscible organic solvents) is coarsely emulsified in an aqueous medium. Subsequently, at least a portion of the solvent system is stripped off using elaborate procedures to form a suspension of liposomes.

It is to be noted that the prior art techniques require immiscible, toxic solvents which have to be removed from the system. Furthermore, energy has to be applied to form liposomes after solvent evaporation.

EP-B-0158441 describes a "proliposome" approach which has been developed to facilitate the wider use of liposomes. Proliposomes are defined as progenitors of liposomes and are bilayer lipid compositions that rearrange to form stable liposome vesicles upon the simple addition of excess water. Biologically active compounds present in the system are trapped with high efficiency during the reorganisation into stable vesicles. Under suitable conditions, the conversion of proliposomes to liposomes can take place on moist skin, or mucosa, by utilising the indigenous water present. However, this process can be slow and requires sufficient water to maximise conversion to liposomes.

The present invention therefore seeks to provide an alternative approach for the formation of liposomes or bilayered structures (which terms as used hereafter are to be understood as including films formed by the collapse of liposomes or bilayered structures), that does not necessarily involve addition of excess water, and which is particularly suitable for the "in situ" production of liposomes or bilayered structures on biological surfaces or substrates. The compositions of this invention may therefore be regarded as a form of "proliposomes" in that they are progenitors of liposomes. Moreover, the formation of the liposomes or bilayered structures occurs spontaneously and does not require any form of energy input or mechanical processing.

According to the present invention there is provided a method of forming liposomes or bilayered structures which comprises removing a proportion of the organic liquid from a composition comprising:-
a) at least one bilayer forming lipid;
b) at least one water-miscible organic liquid;
   and
c) a hydrophilic medium,
   with the proviso that water-immiscible organic solvents are substantially absent from the composition, and whereupon the composition spontaneously forms liposomes or bilayered structures.

The present invention further provides a composition comprising a uniform mixture of:-
a) at least one bilayer forming lipid;
b) at least one water-miscible organic liquid;
   and
c) a hydrophilic medium,
   the lipid being present in an amount of from 0.1 to 5.0% by weight of the weight of component b), with the proviso that water-immiscible organic solvents are substantially absent from the composition, and which, upon the removal of a proportion of component b), spontaneously forms liposomes or bilayered structures.

In a particularly preferred embodiment, the composition is placed in contact with an external biological surface, such as human or animal skin or hair, the scalp, cuticles or the surface of a plant, at the time that a proportion of the organic liquid is removed. For example, the composition may be sprayed, poured, applied with a finger or hand, or by any other means, on to the biological surface or substrate. It is to be appreciated, however, that in principle the substrate may be any type of surface. For example, the composition could be applied to a sheet of plastic or any other surface to support a film capable of mimicking a natural membrane.

The formation of liposomes or bilayered structures occurs spontaneously upon the removal of a proportion of the organic liquid of component b) from the composition. It will be appreciated that the amount of organic liquid removed will depend to a large extent upon the formulation of the specific composition concerned, particularly the choice of bilayer forming lipid or lipids, and the circumstances of its use. The proportion of organic liquid removed/remaining can be defined as that which produces the spontaneous formation of liposomes or bilayered structures, and can be determined accordingly. In some compositions it may be necessary to remove a large amount of organic liquid to cause conversion to liposomes, while other compositions may already be on the threshold of conversion and require the removal of a much smaller quantity of organic liquid in order for liposomes to form on a substantial scale. Typically, removal of from about 5% to 50% by weight of the organic liquid results in the formation of large numbers of liposomes or bilayered structures.

The removal of the organic liquid may be effected by any means, such as conversion to esters or other derivatives. Most conveniently, it occurs through evaporation. In many instances, sufficient evaporation will occur naturally at ambient temperature. If desired, however, the process of evaporation can be assisted or accelerated by a current of air or other gas and/or by elevated temperature. When the composition is applied to skin, body heat and/or movement of the subject will promote the evaporation of the organic liquid. At equilibrium there should be sufficient water/moisture to maintain the presence of the liposomes or bilayered structures.

The lipid component may comprise any bilayer forming compounds, including natural, hydrogenated and synthetic phospholipids, glycolipids, long chain dialkyl dimethyl ammonium compounds, mono and dialkyl polyoxyethylene derivatives and polyglycerol esters. The lipid is typically present in an amount of from 0.1 to 5.0% by weight of the weight of the organic liquid component, and more preferably from 1.0 to 5.0%. This level of lipid has the advantage of producing a composition with a texture that is not too sticky and also results in the formation of discrete liposomes or bilayered structures. Higher proportions of lipid, however, tend to result in the formation of bilayered structures in a more tightly packed arrangement (e.g. a film).

In addition, the composition may also contain other natural and synthetic lipids which modify the melting point, phase transition temperature and charge of the bilayered lipid. Advantageously, though not necessarily, the lipid component comprises a mixture of a neutral lipid (such as phosphatidylcholine) and a charged lipid (such as phosphatidic acid). Typically, the ratio of neutral lipid to charged lipid should be within the range of from 5:1 to 20:1. It has been found that the presence of the charged lipid increases the number and improves the quality of liposomes formed by the composition.

The composition also includes at least one water-miscible organic liquid and which may, though need not necessarily, be a solvent for the lipid component. Typically, the organic liquid is present in the composition in an amount such that the proportion by weight of the bilayer forming lipid to said organic liquid is from 0.1 to 5.0%. Preferably, a volatile organic liquid having a higher vapour pressure (i.e. a lower boiling point) than water is chosen. Where the compositions of the invention are intended for pharmaceutical use, the organic liquid will usually need to be non-toxic. Most preferably, it comprises an aliphatic alcohol such as glycerol, propylene glycol or, most particularly, ethanol. Isopropyl alcohol, methanol, butanol and ethylene glycol may also be used in appropriate circumstances. Combinations of organic liquids, especially mixtures of ethanol and glycerol or propylene glycol, may be used. Although glycerol and propylene glycol (particularly in combination with ethanol) may be considered as a solvent for the lipid, they are also appropriately hydrophilic solutions.

In this specification the hydrophilic medium comprises water (which term is to be understood as covering any aqueous liquid containing buffers, salts or dissolved compounds), glycerol, propylene glycol, a polyethylene glycol, or a mixture of two or more thereof. Preferably, it comprises a mixture of water and glycerol or water and propylene glycol in equal parts by weight. The hydrophilic medium is initially present in the composition in an amount less than that which causes the spontaneous formation of liposomes or bilayered structures. Such a composition, which may be referred to as a proliposome or volatile proliposome composition, is normally clear or translucent in appearance since it contains few liposomes. Typically, the proportion of the lipid component to the hydrophilic medium in the composition will be within the range of from 1:1 to 1:40, preferably from 1:2 to 1:20 and more preferably from 1:4 to 1:10. Depending upon the nature and proportions of the other components present, the composition could contain the hydrophilic medium in an amount of up to about 70% by weight and still remain clear or only slightly translucent. Some liposomes are present in such compositions. More typically, however, the hydrophilic medium will be present in an amount of up to about 40% by weight.

In practice, a humectant may advantageously be included to control evaporation of the hydrophilic medium and ensure that there is some hydrophilic medium present. Any material having humectant properties could be used for this purpose and would be employed in conventional amounts. Where the humectant is a solid substance (for example, a sugar such as sucrose, sorbitol or mannitol, or a polyol), it is usually necessary for the hydrophilic medium to include at least a proportion of water. Conveniently, glycerol, propylene glycol and polyethylene glycols can themselves be considered as humectants and no other humectant materials may be needed in compositions containing these substances.

Surprisingly, the hydrophilic medium may be composed entirely of glycerol, propylene glycol or a polyethylene glycol, or a mixture of such materials. In a preferred embodiment, however, the hydrophilic medium comprises a mixture of water and glycerol or water and propylene glycol. Most preferably, the mixture contains equal amounts (i.e. 50% by weight) of each component. It has also been found that the presence of a proportion of glycerol and/or propylene glycol in the hydrophilic medium improves the stability of the composition. This ensures that the preparation remains on a substrate as liposomes or bilayered structures.

The method and composition of this invention will have uses in various fields, including pharmaceutical preparations for topical administration, diagnostic preparations, cosmetics and toiletries, insecticides and horticultural preparations and appliances. In such applications, the composition may include one or more "biologically active compounds" in solution or suspension. This term is to be understood as including medicaments (human and veterinary), anti-bacterial, anti-microbial and anti-fungal agents, anti-inflammatory agents, steroids, proteins, enzymes, hormones, vitamins, marker compounds, plant and herbal extracts, essences, pheromones and fragrances within its definition, but is not restricted thereto Other possible ingredients would include preservatives and anti-oxidants to extend the life of the product.

One particularly interesting area of use is in cosmetics and toiletry preparations. The composition would typically be sprayed or applied on to the skin and, after a period of perhaps 15 to 30 seconds, during which a proportion of the organic liquid evaporates off, would spontaneously form liposomes or bilayered structures on the surface of the skin. A fragrance entrapped within the liposomes on the skin's surface would be released gradually over a period of time, thus giving a "sustained release" perfume product. Application of fragrances in this way should also reduce the problems of skin irritation which can arise.

The composition of this invention may be presented in a variety of different forms, including as a gel or as a solid stick. The latter form is particularly appropriate for deodorant/anti-perspirant toiletry preparations.

The present invention will now be further illustrated by the following Examples.

### EXAMPLE 1

### Method

Compositions according to the invention were prepared as follows:-

| Formulation Number | Formulation (parts by weight) | | |
|---|---|---|---|
| | Lipid | Organic liquid | Water |
| 1A | 4 | 80 | 16 |
| 1B | 4 | 80 | 16 |
| 2A | 2 | 80 | 18 |
| 2B | 2 | 80 | 18 |
| 3A | 2 | 60 | 38 |
| 3B | 2 | 60 | 38 |
| 4A | 2 | 40 | 58 |
| 4B | 2 | 40 | 58 |

The lipid component consisted of either Lipid A (Epikuron 180) or Lipid B (Epikuron 145) and the compositions are identified accordingly, i.e. as 1A, 1B, 2A, 2B, etc. The organic liquid was ethanol.

The compositions were prepared by initially dissolving the lipid in ethanol followed by the addition of distilled water (demineralised or purified water could also be used).

### Results

The compositions were placed in an uncovered petri dish and held at a temperature of 32°C in a humid atmosphere. This temperature was chosen to approximate skin temperature and facilitate the evaporation process (but it is not in itself necessary for the formation of liposomes).

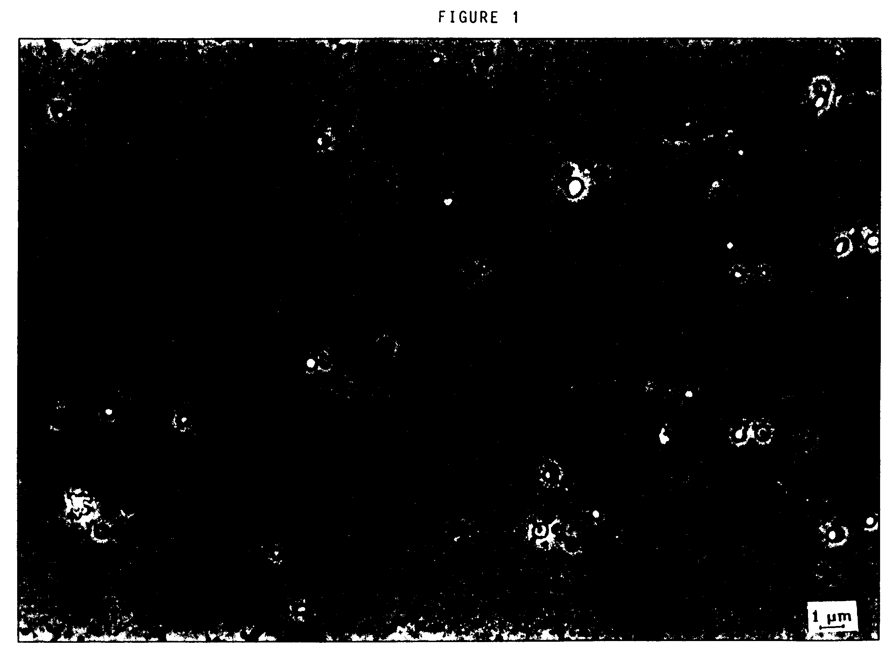

Formulation 2A was initially clear, indicating an absence of liposomes. After 1 hour, the alcohol content had fallen to 67% but there was no visible formation of liposomes. After 2 hours, the alcohol content was down to 38% and a substantial number of liposomes had formed (as shown in Figure 1).

Formulation 2B was initially clear, indicating that no liposomes were present. After 1 hour, the alcohol content had fallen to 66% but no liposomes were detectable. After 2 hours, the alcohol content was 55% and a substantial number of liposomes could be detected.

Similar studies with Formulations 1A and 1B, 3A and 3B and 4A and 4B also demonstrated that liposomes were formed upon a reduction in the level of the organic solvent present in the compositions.

Formulation 2 was repeated using Epikuron 200. Initially, the formulation was clear indicating that no liposomes were present. After 1 hour, the concentration of alcohol had dropped to approximately 68% with very little liposome formation. After 2 hours, the alcohol content dropped to 48% and a matrix arrangement of bilayered structures was obtained.

In each of these formulations, after approximately 3-4 hours a film was obtained comprising collapsed liposomes or bilayered structures.

### EXAMPLE 2

### Method

Compositions according to the invention were prepared as follows:-

| Formulation Number | Formulation (parts by weight) | | | |
|---|---|---|---|---|
| | Lipid | Organic liquid | Water | Glycerol (Propyleneglycol) |
| 5 | 2(PC) | 80 | 18 | 0 |
| 6 | 2(PC) | 80 | 9 | 9 |
| 7 | 2(PC) | 80 | 0 | (18) |
| 8 | 2(PC/PA) | 80 | 18 | 0 |
| 9 | 2(PC/PA) | 80 | 9 | 9 |
| 10 | 2(PC/PA) | 80 | 0 | (18) |

In Formulations 5, 6 and 7, the lipid component consisted of pure phosphatidylcholine (PC). In each of Formulations 8, 9 and 10, a mixture of 10 parts phosphatidylcholine to 1 part phosphatidic acid (PA) was used. In all cases, the organic liquid was ethanol.

The compositions were prepared by dissolving the lipid in ethanol. The hydrophilic medium consisting of water, water/glycerol or propylene glycol was added to obtain the clear proliposome.

### Results

The compositions were placed in an uncovered petri dish and held at a temperature of 32°C in a humid atmosphere for approximately 2-3 hours or until large numbers of liposomes were formed. Reference is directed to the accompanying drawings, in which:

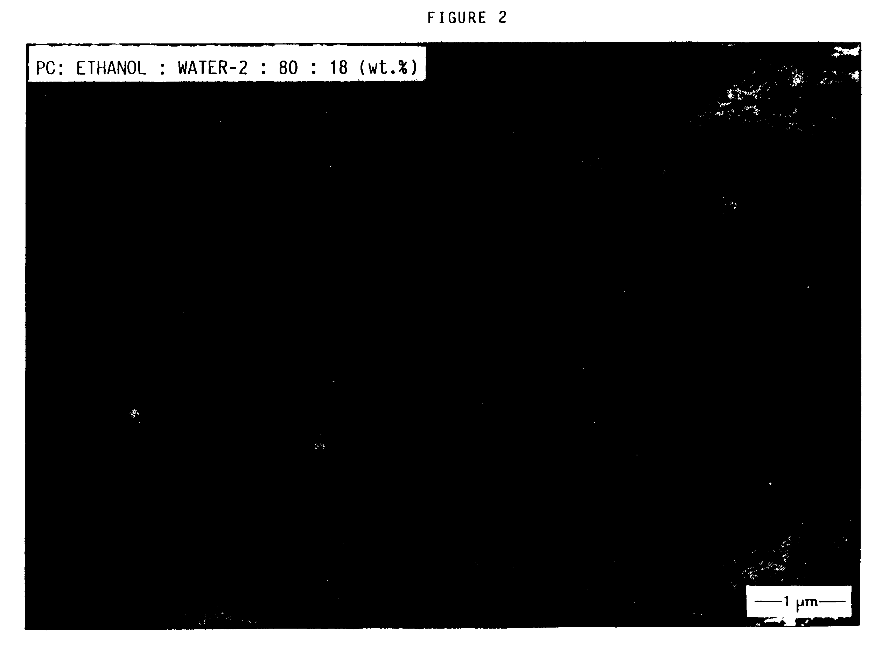

- Figure 2 is a freeze-fracture electron micrograph showing Formulation 5 (in which the hydrophilic medium consists of water). A number of liposomes and related bilayer structures have formed.

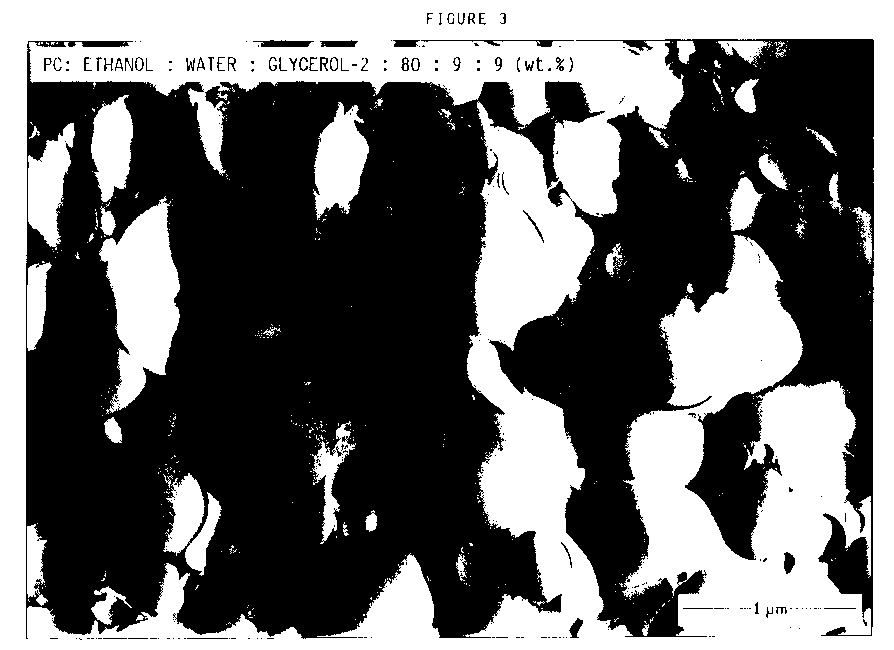

- Figure 3 is a freeze-fracture electron micrograph showing Formulation 6 (in which the hydrophilic medium consists of 50% water and 50% glycerol). A large number of well-defined liposomes are clearly visible.

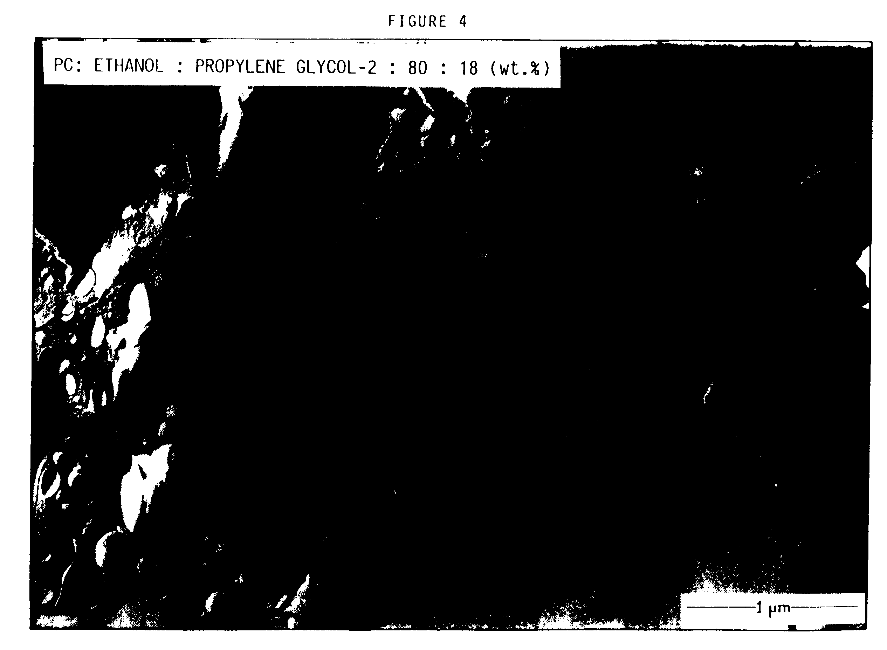

- Figure 4 is a freeze-fracture electron micrograph showing Formulation 7 (in which the hydrophilic medium consists of propylene glycol alone). Some liposomes have formed, but substantially fewer than in Figure 3.

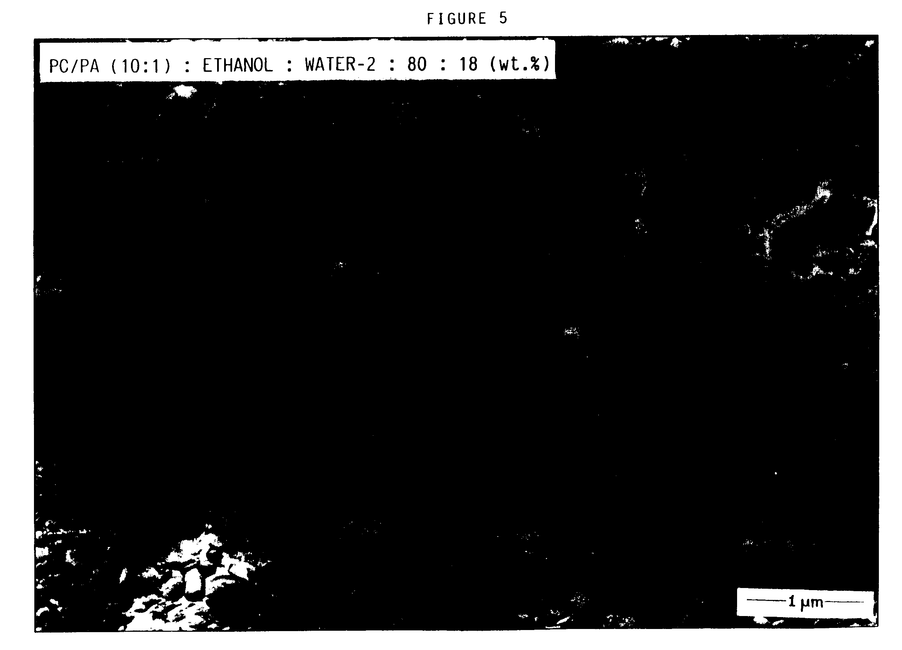

- Figure 5 is a freeze-fracture electron micrograph showing Formulation 8 (in which the lipid component is a mixture of PC and PA, while the hydrophilic medium is water). Rather more liposomes have formed than in Figure 2, illustrating the advantageous effect of including a proportion of a charged lipid (PA) in the composition.

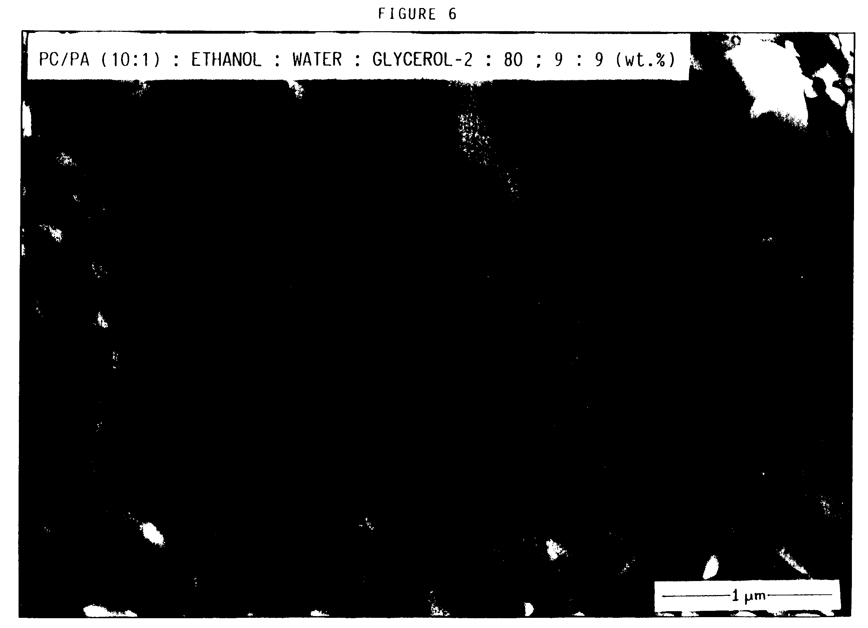

- Figure 6 is a freeze-fracture electron micrograph showing Formulation 9 (in which the lipid component is a mixture of PC and PA, while the hydrophilic medium contains 50% water and 50% glycerol). A large number of well-formed liposomes have been formed, illustrating the advantageous effects of these features of a particularly preferred composition of this invention.

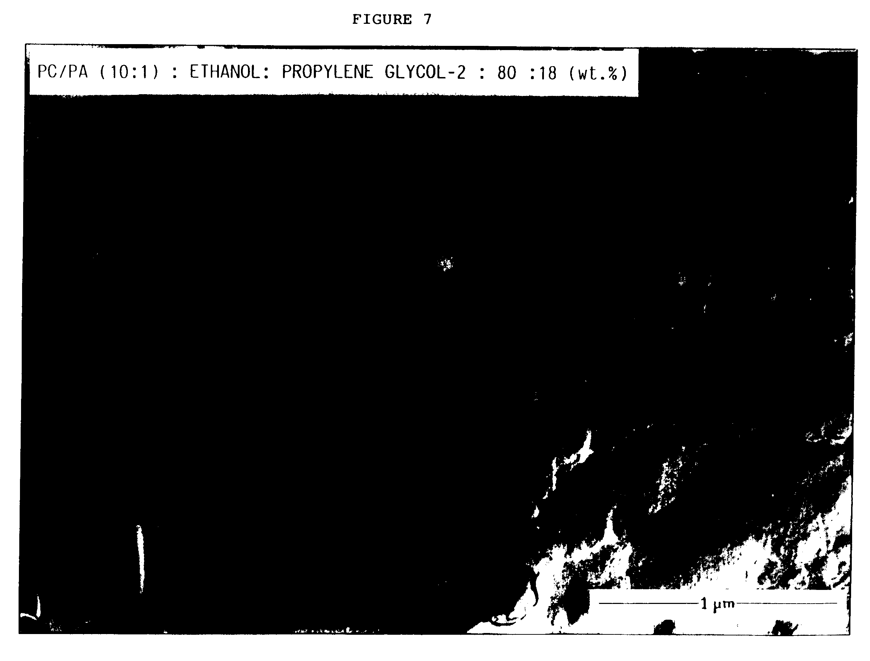

- Figure 7 is a freeze-fracture electron micrograph showing Formulation 10 (in which the lipid component is a mixture of PC and PA, while the hydrophilic medium consists of propylene glycol alone). Numerous distinct liposomes have formed.

The sequence resulting in liposome formation is thought to be triggered by the loss of ethanol from a typical system containing lipid/ethanol/water/glycerol.

The composition is normally clear, in which case the lipid is in true molecular solution or in the form of spherical micelles. It may also be a translucent mixture containing micelles and bilayers, depending on the amount of water/glycerol. Loss of ethanol from the system (e.g. by evaporation) leaves behind a composition where the proportion of water/glycerol increases. Progressively, more of the lipid tends to reorganise into liposomes and bilayered structures. A biologically active compound included in the composition will be partitioned into the bilayers. The inclusion of glycerol ensures that the external phase does not dry out completely. It can be seen from the electron micrographs that the majority of liposomes formed by this method are discrete and in the region of about 200nm. The method is extremely efficient, gives high levels of entrapment and does not require energy input.

### EXAMPLE 3

A composition according to the present invention was prepared in the form of a gel from the following materials. All parts are by weight.

| | % |
|---|---|
| Lipid (Epikuron 180) | 4 |
| Ethanol | 52.5 |
| Distilled Water | 36 |
| Glycerol | 7 |
| Carbopol 940 | 0.5 |

The lipid was dissolved in ethanol at room temperature. Carbopol 940 (carbopol is a trade mark) was dissolved in water/glycerol and gelled with triethanolamine at about pH7. Lipid-soluble compounds may be dissolved in the ethanol/lipid solution, while water-soluble compounds may be dissolved in the water/glycerol solution. Paraffins, volatile silicones, oils, fats and waxes, provided that they do not affect the stability of the liposomes, may be dissolved/dispersed in the composition. The two components were combined to form a proliposome gel.

In place of Carbopol 940, a gel-forming material, other hydrocolloids (such as cellulose gums or natural gums, e.g. xanthan gums) may be used.

It is envisaged that a composition of this type containing a biologically active ingredient would be particularly useful for cosmetic and pharmaceutical applications. For example, inclusion of a drug such as ibuprofen could provide a topical pain relief formulation for sufferers of rheumatism in a form which is easily applied to the affected areas of the body. Furthermore, an active ingredient entrapped within the liposomes on the skin's surface would be released gradually over a period of time and so produce a sustained release effect.

### EXAMPLE 4

A composition according to the present invention was prepared in the form of a solid stick using the following materials. All parts are by weight.

| | % |
|---|---|
| Lipid (Epikuron 180) | 4 |
| Ethanol | 74.5 |
| Distilled Water | 8 |
| Propylene glycol | 8 |
| Lauric acid diethanolamide | 1 |
| Sodium stearate | 4.5 |

The lipid was dissolved in 20 parts of ethanol. Sodium stearate and lauric acid diethanolamide were dissolved in the remaining ethanol and propylene glycol at about 45°C in a closed vessel. As in Example 3, various paraffins, volatile silicones, oils, fats and waxes may be included to impart emolliency. The two solutions were combined and water was added. The resultant solution was maintained at 45°C and poured into moulds which solidified upon cooling into sticks.

It is envisaged that a composition of this type would be particularly useful as a deodorant/anti-perspirant toiletry product for application to the body. Furthermore, a fragrance entrapped within the liposomes on the skin's surface would be released gradually over a period of time and so produce a sustained release effect.

### EXAMPLE 5

A composition according to the present invention was prepared using the following materials. All parts are by weight.

| | % |
|---|---|
| Lipid (Epikuron 200SH) | 3 |
| Propylene glycol | 20 |
| Ethanol | 77 |

The hydrogenated lipid was dissolved in the ethanol at about 45°C. Propylene glycol was added to obtain a homogeneous solution. At room temperature, the composition was somewhat turbid (indicating the presence of some liposomes). However, large numbers of liposomes were only formed upon evaporation of the ethanol.

### EXAMPLE 6

A concentrate was prepared from the following materials. All parts are by weight.

| | % |
|---|---|
| Lipid (Epikuron 200) | 15 |
| Ethanol | 10 |
| Glycerol | 75 |

This concentrate was then diluted (5 fold) with 80g of a solution of 80% ethanol/20% water, so as to produce a composition of the present invention containing:-

| | % |
|---|---|
| Lipid | 3 |
| Glycerol | 15 |
| Ethanol | 66 |
| Water | 16 |

A biologically active ingredient and auxiliary emollients could be added at this stage and the composition is then ready for use in the usual manner. The active ingredient may be dissolved or suspended.

It is envisaged that the compositions could conveniently be manufactured, transported and stored in the form of a concentrate and then diluted prior to sale.

## Claims

1. A method of forming liposomes or bilayered structures which comprises removing a proportion of the organic liquid from a composition comprising:-
a) at least one bilayer forming lipid;
b) at least one water-miscible organic liquid;
and
c) a hydrophilic medium,
with the proviso that water-immiscible organic solvents are substantially absent from the composition, and whereupon the composition spontaneously forms liposomes or bilayered structures.

2. A method as claimed in claim 1, wherein the hydrophilic medium comprises water, glycerol, propylene glycol, a polyethylene glycol or a mixture of two or more thereof.

3. A method as claimed in claim 1 or claim 2, wherein the composition is placed in contact with a biological surface.

4. A method as claimed in any of the preceding claims, wherein the removal of the organic liquid occurs through evaporation.

5. A method as claimed in any of the preceding claims, wherein the lipid of component a) of the composition is present in an amount of from 0.1 to 5.0% by weight of the weight of component b).

6. A method as claimed in any of the preceding claims, wherein the proportion of component a) to component c) in the composition is within the range of from 1:1 to 1:40 parts by weight.

7. A method as claimed in any of the preceding claims, wherein the composition also includes a biologically active compound.

8. A composition comprising a uniform mixture of:-
a) at least one bilayer forming lipid;
b) at least one water-miscible organic liquid;
and
c) a hydrophilic medium,
the lipid being present in an amount of from 0.1 to 5.0% by weight of the weight of component b), with the proviso that water-immiscible organic solvents are substantially absent from the composition, and which, upon the removal of a proportion of component b), spontaneously forms liposomes or bilayered structures.

9. A composition as claimed in claim 8, wherein the hydrophilic medium comprises water, glycerol, propylene glycol, a polyethylene glycol or a mixture of two or more thereof.

10. A composition as claimed in claim 8 or claim 9, wherein the hydrophilic medium comprises a mixture of water and glycerol or water and propylene glycol in equal parts by weight.

11. A composition as claimed in any of claims 8 to 10, wherein the proportion of component a) to component c) in the composition is within the range of from 1:1 to 1:40 parts by weight.

12. A composition as claimed in any of claims 8 to 11, wherein component a) comprises a mixture of neutral lipid and charged lipid.

13. A composition as claimed in any of claims 8 to 12, wherein component b) is at least one volatile water-miscible organic liquid.

14. A composition as claimed in any of claims 8 to 13, wherein the organic liquid is ethanol, methanol or isopropyl alcohol.

15. A composition as claimed in any of claims 8 to 14, wherein there is also present a biologically active compound.

16. A composition as claimed in claim 15, wherein said biologically active compound comprises a fragrance.

17. A composition as claimed in any of claims 8 to 16, presented in the form of a gel or a solid stick.

18. A concentrate which forms a composition as claimed in any of claims 8 to 17 upon dilution.

19. Liposomes or bilayered structures, or films formed by the collapse of liposomes or bilayered structures, which have been formed by a method as claimed in any one of claims 1 to 7 and/or from a composition as claimed in any one of claims 8 to 17.

## Patentansprüche

1. Eine Vorgangsweise, in der Liposomen oder doppellagige Strukturen gebildet werden, die daraus besteht, daß ein Teil der organischen Flüssigkeit aus der Zusammensetzung entfernt wird, und diese besteht aus;
a) mindestens einem doppellagen-bildendem Lipid;
b) mindestens einem mit Wasser mischbaren organischen Lipid;
und
c) einem wasserliebenden Lösungsmittel
mit dem Vorbehalt, daß die wasser-unmischbaren organischen Lösungsmittel im wesentlichen nicht in der Zusammensetzung vorhanden sind und wonach die Zusammensetzung spontan Liposomen oder doppellagige Strukturen bildet.

2. Eine Verfahrensweise laut Anspruch in Anspruch 1, in der das wasserliebende Lösungsmittel aus Wasser, Glycerol, Propylenglykol, einem Polyäthylenglykol oder einer Mischung aus zwei oder mehr dieser Lösungsmittel besteht.

3. Eine Verfahrensweise laut Anspruch in Anspruch 1 oder Anspruch 2, in der die Zusammensetzung in Kontakt mit einer biologischen Oberfläche gebracht wird.

4. Eine Verfahrensweise laut Anspruch in einem der obigen Ansprüche, in der das Entfernen der organischen Flüssigkeitslösung durch Verdunsten entsteht.

5. Eine Verfahrensweise laut Anspruch in einem der obigen Ansprüche, in der das Lipid aus Bestandteil a) der Zusammensetzung mit einer Menge von 0,1 bis 5,0% nach Gewicht des Gewichts der Zusammensetzung b) vorhanden ist.

6. Eine Verfahrensweise laut Anspruch in einem der obigen Ansprüche, in der der Anteil des Bestandteils a) zu Bestandteil c) in der Zusammensetzung innerhalb des Bereichs von 1:1 bis 1:40 Teilen nach Gewicht beträgt.

7. Eine Verfahrensweise laut Anspruch in einem der obigen Ansprüche, in der die Zusammensetzung auch eine biologisch aktive Verbindung enthält.

8. Eine Zusammensetzung, die eine gleichmäßige Mischung enthält aus:
a) mindestens einem doppellagen-bildenden Lipid;
b) mindestens einer wassermischbaren, organischen Flüssigkeit
und
b) einem wasserliebenden Lösungsmittel
Dem vorhandenen Lipid in einer Menge von 0,1 bis 5,0% nach Gewich des Gewichts des Bestandteils b), mit dem Vorbehalt, das wasser-unmischbare, organische Lösungsmittel in der Zusammensetzung im wesentlichen nicht vorhanden sind und die, nach Entfernen von Bestandteil b), Liposomen oder doppellagige Strukturen spontan bilden.

9. Eine Zusammensetzung laut Anspruch in Anspruch 8, in der das wasserliebende Lösungsmittel Wasser, Glycerol, Propylenglykol, ein Polyäthylenglykol oder eine Mischung aus zwei oder mehr dieser besteht.

10. Eine Zusammensetzung laut Anspruch in Anspruch 8 oder Anspruch 9, in der das wasserliebende Lösungsmittel aus einer Mischung aus Wasser und Glycerol oder aus Wasser und Propylenglykol zu gleichen Teilen nach Gewicht besteht.

11. Eine Zusammensetzung laut Anspruch in einem Anspruc von 8 bis Anspruch 10, in der der Anteil des Bestandteils a) bis Bestandteil c), in der Zusammensetzung innerhalb des Bereichs von 1:1 bis 1:40 Teilen nach Gewicht liegt.

12. Eine Zusammensetzung laut Anspruch in einem Anspruch von 8 bis Anspruch 11, in der der Bestandteil a) aus einer Mischung aus einem neutralen Lipid und einem geladenen Lipid besteht.

13. Eine Zusammensetzung laut Anspruch in einem Anspruch von 8 bis Anspruch 12, in der der Bestandteil b) mindestens eine volatile wassermischbare, organische Flüssigkeit enthält.

14. Eine Zusammensetzung laut Anspruch in einem Anspruch von 8 bis Anspruch 13, in der die organische Flüssigkeit Äthanol, Methanol oder Isopropylalkohol ist.

15. Eine Zusammensetzung laut Anspruch in einem Anspruch von 8 bis Anspruch 14, in der auch eine biologisch aktive Verbindung vorhanden ist.

16. Eine Zusammensetzung laut Anspruch in Anspruch 15, in der die erwähnte biologisch aktive Zusammensetzung einen Duftstoff enthält.

17. Eine Zusammensetzung laut Anspruch in einem Anspruch von 8 bis Anspruch 16, die in Form eines Gel oder eines festen Stifts präsentiert wird.

18. Ein Konzentrat, das nach dem Verdünnen eine Zusammensetzung laut Anspruch in einem Anspruch von 8 bis Anspruch 17 bildet.

19. Liposomen oder doppellagige Strukturen oder Schichten, die durch das Scheitern von Liposomen oder doppellagigen Strukturen gebildet wurden, die laut einer Verfahrensweise laut Anspruch in einem Anspruch 1 bis Anspruch 7 und/oder aus einer Zusammensetzung laut einem Anspruch von 8 bis 17 gebildet wurde.

## Revendications

1. Une méthode pour former des liposomes ou structures à double couche qui comprend le retrait d'une proportion du liquide organique de la préparation comprenant:
a) au moins un lipide formant une double couche
b) au moins un liquide organique soluble dans l'eau.
et
c) un milieu hydrophile,
avec la condition que les solvants organiques insolubles dans l'eau soient absents en grande partie dans la préparation, et de ce fait la préparation forme spontanément des liposomes ou structures à double couche.

2. Une méthode comme révendiquée dans la révendication 1. dans laquelle le milieu hydrophile comprend de l'eau, du glycérol, du propylène glycol, un polyethylene glycol ou un mélange de deux ou plus de ces derniers.

3. Une méthode comme reveniquée dans la révendication 1. ou 2., dans laquelle la préparation est plaçée en contact avec une surface biologique.

4. Une méthode comme revendiquée dans n'importe quelles des revendications précédentes, dans laquelle le retrait du liquide organique a lieu en s évaporant.

5. Une méthode comme revendiquée dans n'importe quelles des revendications précédentes, dans laquelle le lipide d'un composant a) de la préparation est présent dans une quantité d'à peu près 0.1 à 5.0% en poids du poids du composant b).

6. Une méthode comme revendiquée dans n'importe quelles des revendications précédentes, dans laquelle la proportion du composant a) au composant c) dans la préparation est dans la marge de 1:1 à 1:40 mesures en poids.

7. Une méthode comme revendiquée dans n'importe quelles des revendications précédentes, dans laquelle la préparation inclut aussi un composé biologiquement actif.

8. Une préparation comprenant un mélange uniforme d':
a) au moins un lipide formant double couche
b) au moins un liquide organique soluble dans l'eau.
et
c) un milieu hydrophile,
Le lipide étant présent dans une quantité de 0.1 à 5.0% en poids du poids du composant b) avec la condition que les solvants organiques insolubes dans l'eau soient absents en grande partie de la préparation, et que, lors du retrait d'une proportion du composant b) forme spontanément des liposomes ou structures à double couche.

9. Une préparation comme révendiquée dans la révendication 8. dans laquelle le milieu hydrophile contienne de l'eau, du glycérol, du propylène glycol, un polyethyléne glycol ou un mélange de deux ou plus de ces derniers.

10. Une préparation comme révendiquée dans la révendication 8. ou 9., dans laquelle le milieu hydrophile comprend un mélange d'eau, ou glycérol, ou d'eau et de propylène glycol, en mesures égales en poids.

11. Une préparation comme révendiquée dans la révendication 8. à 10., dans laquelle la proportion de composant a) au composant c) dans la préparation est dans la marge de 1:1 à 1:40 mesures en poids.

12. Une préparation comme revendiquée dans n'importe quelles des revendications 8 à 12, dans laquelle le composant a) comprend un mélange de lipide neutre et de lipide chargé.

13. Une préparation comme revendiquée dans n'importe quelles des revendications 8 à 12, dans laquelle le composant b) est au moins un liquide organique volatif soluble dans l'eau.

14. Une préparation comme revendiquée dans n'importe quelles des revendications 8 à 13, dans laquelle le liquide organique est de l'éthanol, de l'alcool méthanol ou isopropyl.

15. Une préparation comme revendiquée dans n'importe quelles des revendications 8 à 14, dans laquelle il y a aussi present un composé biologiquement actif.

16. Une préparation comme revendiquée dans n'importe quelles des revendications 15, dans laquelle le composé biologiquement actif dit comprend un parfum.

17. Une préparation comme revendiquée dans n'importe quelles des revendications 8 à 16, presentée sous la forme d'un gel ou bâton solide.

18. Un concentré qui forme une préparation comme revendiqué dans n'importe quelle des revendications 8 à 17 à la dilution.

19. Les liposomes ou les structures à double couche, ou les films (couches très minces) formés par l'affaisement des liposomes ou des structures à double couche qui ont été formés par une méthode comme revendiquée dans n'importe quelles des revendications 1 à 7 et/ou de la préparation comme revendiquée dans n'importe quelles des revendications 8 à 17.
